# EUROPEAN PATENT APPLICATION

(11) **EP 1 970 510 A1**
(43) Date of publication of application: **17.09.2008**
(21) Application number: 07104068.7
(22) Date of filing: 13.03.2007
(51) Int. Cl.: E05B 67/00, E05B 73/00, E05B 45/00

(54) **Security device**

(71) Applicant: MW Security AB, 211 24 Malmö (SE)
(72) Inventor: Nilsson, Thomas, 217 49 Malmö (SE)
(74) Representative: Akerman, Marten Lennart

(57) **Abstract**

The invention relates to a security device (10) for protecting an item from theft, comprising: at least one cable (20) with two ends (20a, 20b), a cable lock (30) that is adapted for releasably attaching the two cable ends together by means of a locking device (40) for forming a loop, the cable lock (30) comprising a first cable end holding part (30a) and a second cable end holding part (30b), which parts are adapted to releasably lock together with the locking device for forming the cable loop. The cable lock (30) comprises a cable adjusting device (50), which is configured for forming an adjustable loop for the cable (20).

## Description

### Technical field of the invention

The invention relates to a device for securing an item. Specifically, the invention relates to an adjustable device where a cable or wire is drawn through clothing, e.g. through the sleeve of a jacket, one leg of a pair of trousers or the sleeve of a sweater and out again, either through the other sleeve of the jacket or sweater, the other leg of the pair of trousers, or through any other opening, e.g. in the form of a fixed suspension opening, or wrapped around the item and then locked together as a cable loop. The security device comprises a cable length adjusting device that is adapted to tighten and widen the cable loop as desired.

### Description of related art

Theft and unauthorized tampering of goods and items for purchase is a constant threat to retail stores. Today, it is very difficult to protect items from being removed by an unauthorized person in shops and malls. For preventing item thefts, i.e. removing of items from the shop without paying for them, the item is provided with an element which trigger an alarm if the item is moved from the shop or a defined area in the mall, or the item, if having no alarm, is fastened by a long cable or wire to a fixed/firm object, e.g. by fastening a wire lock for a bicycle around a bicycle rack or a pole. When protecting items, one must balance the grade of security with cost, accessibility, especially for clothing that is tried on, i.e. a jacket or other clothing provided with an alarm must be easy to try on, and appealing looks, i.e. consumers want to be able to see, and also often want to touch and feel the clothing before buying it. It is well known to use a number of solutions in order to protect items and to make the items accessible at the same time, such as lockable cables with alarms that may be removed when trying on the clothing or alarm pins that are removed from the item at the paydesk. These solutions provides means to protect an item from being removed by an unauthorized person undetected.

However, these solutions have disadvantages in that they require cables with certain dimensions, i.e. lengths, e.g. a long length for securing the alarm to a big item, such as big and thick winter jackets, bicycles or other large items, and a short cable length for securing for example children's clothing, and items with unusual size and/or shape will require cables with a lot of "over dimensioned" length resulting in a unduly handling and manipulation of each cable wasting time and increasing costs for this handling and storing of unused cable locks.

Moreover, these prior art cable locks are difficult to handle as they require complicated manipulation both when attaching the cable to or through the item and when removing the cable from the item as an unnecessary long cable length is difficult to keep track of and control and occupies a lot of space. These known cable alarm locks also require items that have certain sizes and shapes, whereby, e.g. a cable lock with a shorter cable for a small item can not be used on a larger item if the cable locks with longer lengths for the larger items are out of stock. Furthermore, cable locks with unnecessary long wires are easily tangled, both when used and stored for future use, requiring a lot of time and effort to disentangle, and are, in some cases, impossible to disentangle. These prior art cable locks are therefore cumbersome to use and bulky.

### Summary

An overall object of the invention is therefore to provide a simplified construction for a anti-theft or security device which includes at least one cable lock with an adjustable cable allowing for differently sized cable loops to be formed for securing an item to be protected from theft and which is simple to operate, wherein no extra tool is required to adjust the cable loop. It is therefore desired to provide an improved security device that secures items for purchase and is adaptable to different shapes and sizes of the items.

The anti-theft device of the present invention fulfils this object by means of a security device for protecting an item from theft, comprising: at least one cable with two ends, a cable lock that is adapted for releasably attaching the two cable ends together by means of a locking device for forming a loop, the cable lock comprising a first cable end holding part and a second cable end holding part, which parts are adapted to releasably lock together with the locking device for forming the cable loop, characterized in that the cable lock comprises a cable adjusting device, which is configured for forming an adjustable loop for the cable, wherein the first cable end holding part comprising a biased drum rotatably mounted therein and operatively connected to one end of the cable for winding up of the cable, and a cable controlling device operatively connected to the biased drum and operable to narrow the cable loop by releasing the drum for winding up the cable and to brake the unwinding of the cable such that the size of the cable loop is adjusted.

In one embodiment of the security device, one of the cable end holding parts comprises an alarm element adapted for triggering an alarm if the item is removed from a certain defined area.

In another embodiment, the cable end holding parts are movably interconnected, such that the parts are displaceable in opposite directions in relation to each other to allow opening or closing of the cable loop for removing or securing the item when the locking device is released and fixed in relation to each other to close the cable loop for holding and securing the item from theft when the locking device is locked.

In yet another embodiment, one cable end holding part has at least one male member extending in the direction of movement for the two cable end holding parts and facing at least one female member of the other cable end holding part, which female and male members are configured for displaceable interconnection in opposite directions. In still another embodiment, one cable end holding part has two male members extending in the direction of movement for the two cable end holding parts, each male member facing a female member of the other cable end holding part.

A first male member in another embodiment is adapted to guide the movement of the cable end holding parts in relation to each other and to form part of an alarm circuit adapted to trigger an alarm if the cable lock is tampered with. In yet another embodiment, a second male member is adapted to guide the movement of the cable end holding parts in relation to each other such that the stiffness of the cable lock is increased to make the cable lock resistive against tampering.

Moreover, the cable controlling device in one embodiment comprises an operable actuator with a braking member, the actuator being biased so that when in an inoperative position the braking member engages the biased drum to brake the unwinding of the cable and when the actuator is operated the braking member is moved out of engagement with the drum to release the drum for winding up the cable such that the size of the cable loop is adjusted.

In still another embodiment, the locking device in one of the cable end holding parts comprises at least one magnetically attractable leaf spring having an edge portion which in a rested state is engaged with the other cable end holding part.

Furthermore, the cable lock in another embodiment comprises an alarm circuit connected to the cable, configured to sound an alarm when the cable is cut off.

Yet another embodiment of the security device comprises an electronic article surveillance (EAS) tag.

In accordance with the invention, the improved security device for items to be protected from theft, e.g. clothes and bicycles, simplifies the manipulation, handling and assembly of a security device for such items, enhances the degree of security of such security devices, reduces the size of the security device such that it occupies less space, both in use and when stored for future use, and reduces the need of different security devices for different items. Furthermore, the inventive security device is cheap, especially to manufacture, and adaptable, especially to items with different dimensions. The security device reduces the length of cable in a cable lock, i.e. optimizes the used cable length of the cable lock for securing the item to be protected, whereby the fastening and handling are simplified and the associated costs for this reduced, and the storage of the cable locks simplified, i.e. the cable length may be minimized if the cable lock is to be stored for future use. Moreover, by providing the security device with a cable lock having an adjustable cable length, the field of application for the security device is widened enormously as the same cable lock may be used for many differently sized items compared to prior art cable locks where different cable locks are impossible to use for larger items than intended.

### Brief Description of the Drawings

The invention will be described in more detail below with reference to examples of embodiments and with reference to the attached drawings, of which
Fig. 1 is a view showing an embodiment according to the invention;
Fig. 2 is a view of the embodiment in Fig. 1;
Fig. 3 is a perspective view of the embodiment in Fig. 2,
Fig. 4 is an exploded perspective view of the embodiment in Fig. 3,
Fig. 5 is a perspective view of a part of the embodiment in Fig. 4,
Fig. 6 is a view of the part in Fig. 5,
Fig. 7 is a perspective view from another angle of Fig. 1, and
Fig. 8 is a perspective view from yet another angle of Fig. 1.

### Detailed Description of Embodiments

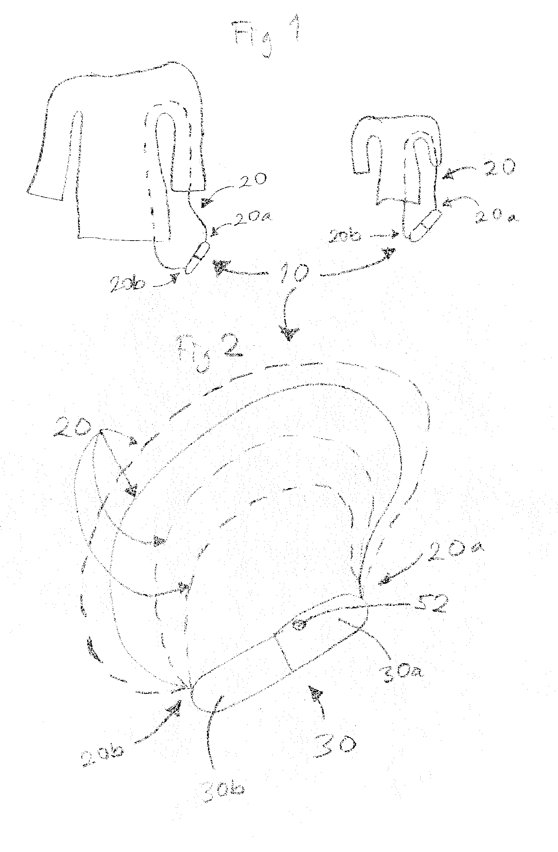

Referring to Figs. 1 to 8, views of an embodiment of the present invention are shown, wherein a security or anti-theft device in general is denoted as 10. In Fig. 1, the same security device 10 is shown in use for protecting two differently sized clothings, an adult's jacket and a childen's jacket. The security device 10 has an elongated shape with rounded ends and comprises at least one cable 20 with two ends 20a, 20b for forming a loop, a larger loop for the bigger jacket to the left in Fig. 1 and a smaller loop for the smaller jacket to the right in Fig. 1.

In Fig. 2, the security device 10 is shown comprising a two-part cable lock 30 having a first cable end holding part 30a and a second cable end holding part 30b, and a cable length adjusting device 52 for narrowing or widening of the cable loop. Here, one size of the cable loop is shown with a solid line while the narrowing and widening of the cable loop are shown with dotted lines.

The security device 10 comprises the cable 20 with two ends 20a, 20b for securing portable items, e.g. jackets as shown in Fig. 1 or other types of clothing or even bicycles (not shown), that may be easily stolen, i.e. removed undetected by unauthorized personnel. The security device 10 in the form of the two-part cable lock 30 also comprises at least one locking device 40. The locking device 40 is arranged at one of the cable ends 20a, 20b, i.e. at one of the cable lock parts 30a, 30b but may be arranged at the other cable end 20a, 20b, i.e. the other cable lock part 30a, 30b instead.

The locking device 40 comprises two spring leaf tongues of metal, as shown in Fig. 2, which are operated by means of an external magnet (not shown) from an engagement and locking state (not shown) where the tongues are in engagement with cavities (not shown) in the first cable end holding part 30a or the second cable end holding part 30b, and a releasing and unlocking state (not shown). This is a known way of locking and unlocking security devices 10, e.g. safers used for CD- and DVD-boxes, and will not be explained in more detail.

The cable lock 30 is adapted for releasably attaching the two cable ends 20a, 20b together by means of the locking device 40 for forming a cable loop in that the first cable end holding part 30a and the second cable end holding part 30b are adapted to releasably lock together with the locking device for forming the cable loop when securing an item to be protected against theft and to be released in relation to each other when releasing the item. The cable lock 30 also comprises a cable adjusting device 50, which is configured for providing an adjustability for the cable loop 20, i.e. its size and shape by widening the cable loop before securing the item and narrowing the cable loop after securing the item and for storage of an unused cable lock, meaning that the length of the cable 20 is adjustable to fit around items with different sizes and shapes and to protect them from theft, or at least make it more difficult to steal them undetected. The first cable end holding part 30a is partly rounded and form a case for enclosing a biased drum 51 being rotatably mounted therein and operatively connected to one end 20a, 20b of the cable 20 for winding up of the cable, i.e. this cable end 20a, 20b is of course fixedly attached to the drum such that the end is secured thereto. The other end 20a, 20b is fixedly attached to the second cable end holding part 30b. In the embodiment shown, the first end 20a of the cable 20 is connected to the first cable end holding part 30a but may instead be connected to the second cable end holding part 30b if the drum 51 instead would be arranged therein.

The drum 51 may be biased by a coiled spring (not shown) such that it is biased for rotary movement in one direction, providing the same function as in a retractable measure tape or the retractable electric cable for a vacuum cleaner and its function and design will not be explained in more detail.

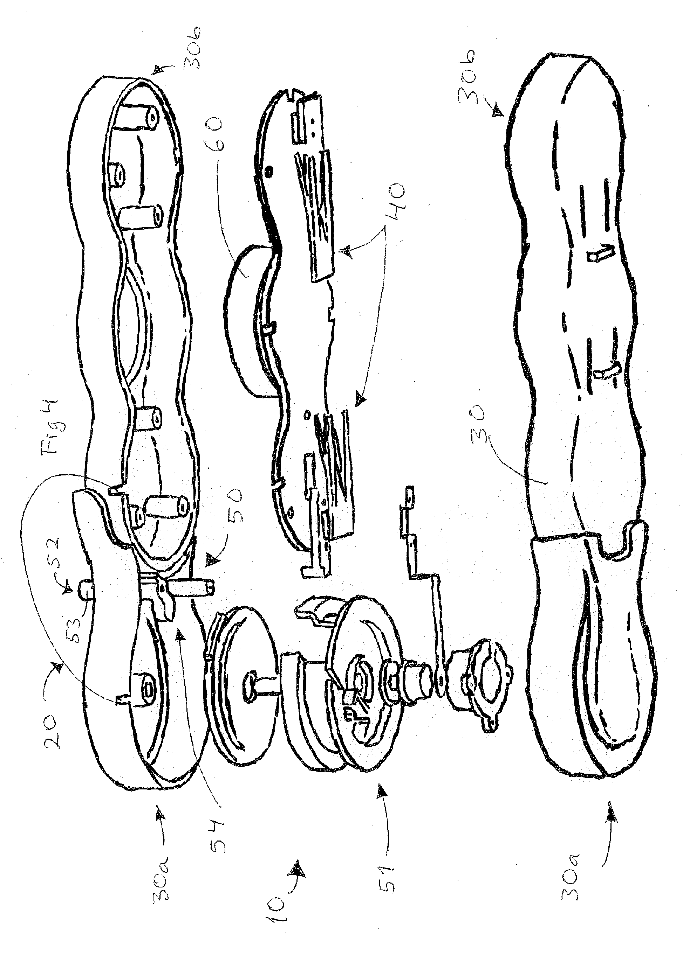

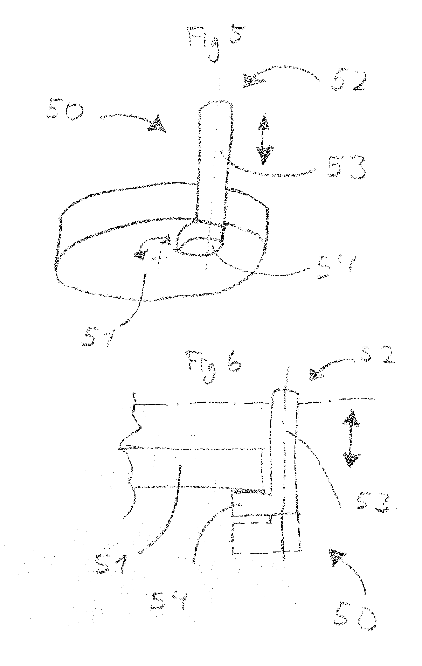

The cable lock 30 in Figs. 1 to 4 comprises a cable controlling/adjusting or retaining/retracting device 50 with an actuator 52 that is operatively connected to the biased drum 51 and operable to narrow the cable loop 20 by releasing the drum for winding up the cable 20 and to brake the unwinding of the cable such that the size of the cable loop is adjusted. This function is achieved in that the cable controlling actuator 52 comprises an operable actuator 53 with a braking member 54 as shown in Figs. 4-6, which actuator is an elongated cylinder-shaped button extending with its longitudinal axis in parallel with the rotational axis of the drum 51. The actuator has two ends, a first upper free end that is protruding out of the cable lock 30 (upwards in Figs. 4-6) to be accessible by a users finger and a lower end (extending downwards in Figs. 4-6) with the braking member 54 extending perpendicularly to the longitudinal axis of the actuator, i.e. in parallel with the plane of the drum, towards the center of the drum. The actuator 53 is biased upwards in Figs. 4-6 to keep the braking member 54 in engagement with the underside of the drum 51 with a suitable force or pressure such that a sufficient and desired braking of the drum is created and the winding of the cable is thereby braked when the actuator is in an inoperative state and when the button of the actuator is manually pressed downwards, i.e. operated, in Figs. 4-6, the braking member 54 is also moved downwards out of engagement and contact with the drum, whereby the drum is released for rotation, i.e. not braked and rewinds, i.e. retracts the cable 20. These two states are shown with solid lines and dotted lines, respectively in Fig. 6. The two-directional arrows in Figs. 5 and 6 show the rotary movement of the drum 51 and the linear movement of the actuator 52. The actuator 52 has the shape of an L in cross-section, as seen in Fig. 6, but may of course be configured differently.

As shown in Figs. 4-6, the braking member engages the periphery of the biased drum 51 to brake the unwinding of the cable 20 and when the actuator 52 is operated manually the braking member is moved out of engagement with the drum for winding up the cable such that the size of the cable loop is adjusted, as desired.

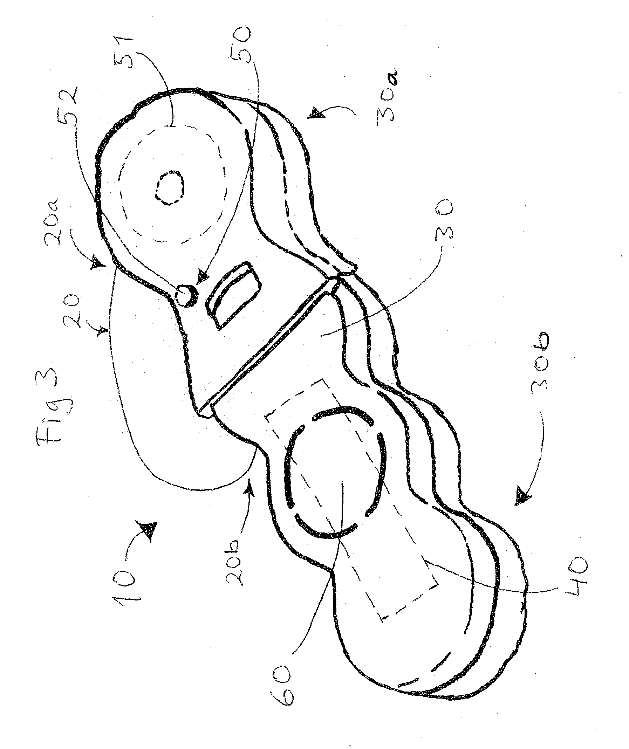

In one embodiment of the security device 10 shown in Fig. 3, one of the cable end holding parts 30a, 30b comprises an alarm element 60 adapted for triggering an alarm if the item is removed from a certain defined area. By providing the security device 10 with an alarm, the protection against an undetected theft is further improved.

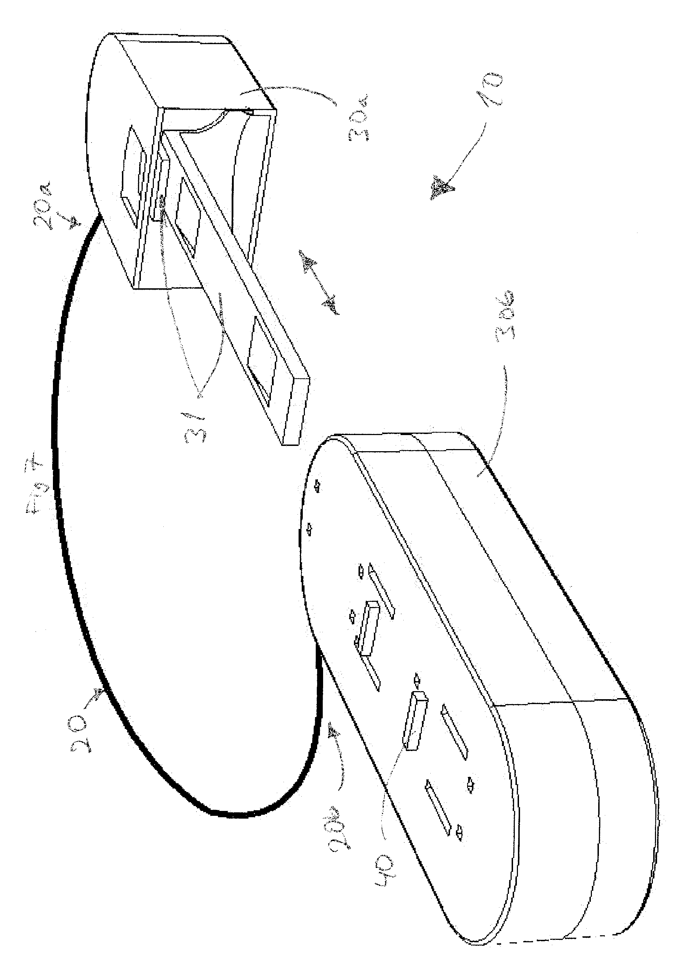

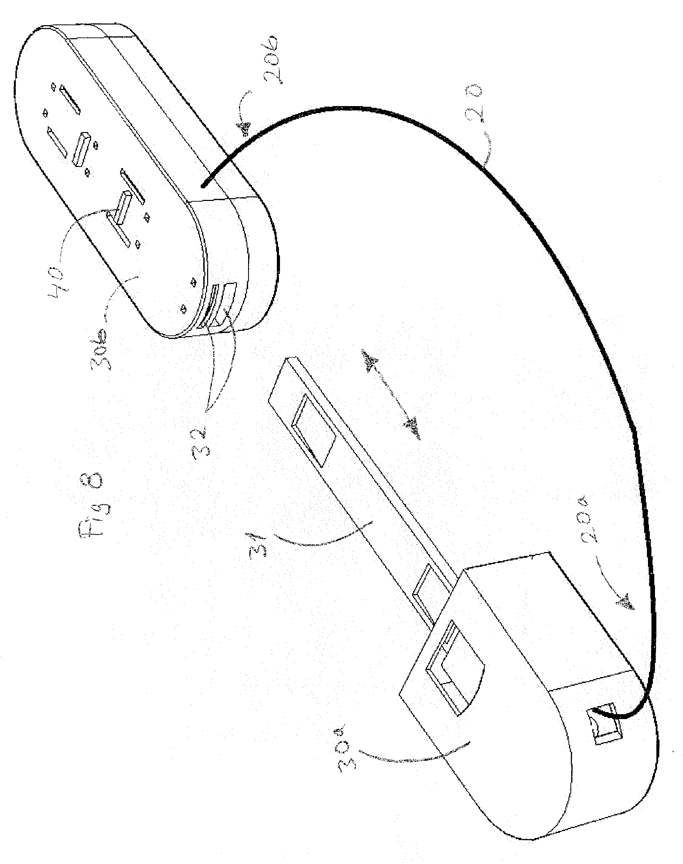

The cable end holding parts 30a, 30b are movably interconnected, such that the parts are displaceable in opposite directions, as shown by the arrow in Figs. 7 and 8, in relation to each other to allow opening or closing of the cable loop 20 for removing or securing the item when the locking device 40 is released and fixed in relation to each other, as shown in Figs. 1-3, to close the cable loop for holding and securing the item from theft when the locking device is locked.

As shown in Figs. 7 and 8, one cable end holding part 30a has at least one male member 31 extending in the direction of movement for the two cable end holding parts 30a, 30b and facing at least one female member 32 of the other cable end holding part 30b (shown in Fig. 8), which members are configured for displaceable interconnection in opposite directions. One cable end holding part 30a in one embodiment has two male members 31 extending in the direction of movement for the two cable end holding parts 30a, 30b, each male member 31 facing a female member 32 of the other cable end holding part 30b (shown in Fig. 7). In another embodiment, a first male member 31 is adapted to guide the movement of the cable end holding parts 30a, 30b in relation to each other and to form part of an alarm circuit adapted to trigger an alarm if the cable lock 30 is tampered with. In yet another embodiment, a second male member 31 is adapted to guide the movement of the cable end holding parts 30a, 30b in relation to each other such that the stiffness of the cable lock 30 is increased to make the cable lock more resistive against tampering, shown in Fig. 3.

The male members 31 have different lengths as shown in Fig. 7, the upper male member 31 is shorter than the lower male member meaning that the stiffness of the cable lock 30 is high when the first cable end holding part 30a and the second cable end holding part 30b are locked together with the locking device 40. This configuration also simplifies the introduction of the male members 31 in the female members 32 as it is impossible to introduce the male members if one of the parts 30a, 30b is turned upside down.

In the security device 10, the locking device 40 in one of the cable end holding parts 30a, 30b comprises at least one magnetically attractable leaf spring 40 having an edge portion which in a rested state is engaged with the other cable end holding part 30a, 30b.

The security device 10 is provided with the cable lock 30 that may comprise an alarm circuit connected to the cable 20, configured to sound an alarm when the cable is cut off.

Moreover, the security device 10 according to the invention may comprise an electronic article surveillance (EAS) tag.

## Claims

1. A security device (10) for protecting an item from theft, comprising: at least one cable (20) with two ends (20a, 20b), a cable lock (30) that is adapted for releasably attaching the two cable ends together by means of a locking device (40) for forming a loop, the cable lock (30) comprising a first cable end holding part (30a) and a second cable end holding part (30b), which parts are adapted to releasably lock together with the locking device for forming the cable loop, **characterized in that** the cable lock (30) comprises a cable adjusting device (50), which is configured for forming an adjustable loop for the cable (20), wherein
the first cable end holding part (30a) comprising a biased drum (51) rotatably mounted therein and operatively connected to one end (20a, 20b) of the cable (20) for winding up of the cable, and
a cable controlling device (52) operatively connected to the biased drum (51) and operable to narrow the cable loop by releasing the drum for winding up the cable (20) and to brake the unwinding of the cable such that the size of the cable loop is adjusted.

2. A security device (10) according to claim 1, wherein one of the cable end holding parts (30a, 30b) comprises an alarm element (60) adapted for triggering an alarm if the item is removed from a certain defined area.

3. A security device (10) according to claim 1 or 2, wherein the cable end holding parts (30a, 30b) are movably interconnected, such that the parts are displaceable in opposite directions in relation to each other to allow opening or closing of the cable loop (20) for removing or securing the item when the locking device (40) is released and fixed in relation to each other to close the cable loop for holding and securing the item from theft when the locking device is locked.

4. A security device (10) according to claim 1, 2 or 3, wherein one cable end holding part (30a) has at least one male member (31) extending in the direction of movement for the two cable end holding parts (30a, 30b) and facing at least one female member (32) of the other cable end holding part (30b), which female and male members are configured for displaceable interconnection in opposite directions.

5. A security device (10) according to claim 4, wherein one cable end holding part (30a) has two male members (31) extending in the direction of movement for the two cable end holding parts (30a, 30b), each male member facing a female member (32) of the other cable end holding part (30b).

6. A security device (10) according to claim 5 with claim 4 dependent on claim 2 or 3, wherein a first male member (31) is adapted to guide the movement of the cable end holding parts (30a, 30b) in relation to each other and to form part of an alarm circuit adapted to trigger an alarm if the cable lock (30) is tampered with.

7. A security device (10) according to claim 5, wherein a second male member (31) is adapted to guide the movement of the cable end holding parts (30a, 30b) in relation to each other such that the stiffness of the cable lock (30) is increased to make the cable lock resistive against tampering.

8. A security device (10) according to any of the preceding claims, wherein the cable controlling device (52) comprises an operable actuator (53) with a braking member (54), the actuator being biased so that when in an inoperative position the braking member engages the biased drum (51) to brake the unwinding of the cable (20) and when the actuator is operated the braking member is moved out of engagement with the drum to release the drum for winding up the cable such that the size of the cable loop is adjusted.

9. A security device (10) according to any of the preceding claims, wherein the locking device (40) in one of the cable end holding parts (30a, 30b) comprises at least one magnetically attractable leaf spring having an edge portion which in a rested state is engaged with the other cable end holding part (30a, 30b).

10. A security device (10) according to any of the preceding claims, wherein the cable lock (30) comprises an alarm circuit connected to the cable (20), configured to sound an alarm when the cable is cut off.

11. A security device (10) according to any of the preceding claims, comprising an electronic article surveillance (EAS) tag.
